# EUROPEAN PATENT SPECIFICATION

(11) **EP 0 224 722 B1**
(45) Date of publication and mention of the grant of the patent: **07.01.1993**
(21) Application number: 86115051.4
(22) Date of filing: 29.10.1986
(51) Int. Cl.: A61K 31/505

(54) **The use of 6H-7,8-Dihydrothiapyrano[3,2-d]pyrimidines for the manufacture of medicaments for use as hypoglycemic agents or as weight reducers for obese patients**
Verwendung von 6H-7,8-Dihydrothiapyrano[3,2d]pyrimidinen zur Herstellung von Arzneimitteln zur Verwendung als hypoglykämische Mittel oder als Abmagerungsmittel für fettsüchtige Patienten
Utilisation des 6H-7, 8-Dihydrothiapyrano[3,2-d]pyrimidines pour obtenir des medicaments utiles comme médicaments hypoglycémiques ou comme agents d'amaigrissement pour des patients obèses

(30) Priority: 04.11.1985 US 794889
(43) Date of publication of application: 10.06.1987
(73) Proprietor: Merck & Co., Inc., Rahway New Jersey 07065-0900 (US)
(72) Inventor: Saperstein, Richard, Edison, N.J. 08820 (US); Tolman, Richard L., Warren, N.J. 07060 (US); Slater, Eve E., Short Hills, N.J. 07078 (US)
(74) Representative: Blum, Rudolf Emil Ernst

(56) References cited:
- FR-A- 2 522 000
- FR-M- 8 437
- CHEM. PHARM. BULL., vol. 34, no. 10, pages 4150-4165; S. OHNO et al.: "Synthesis and Hypoglycemic Activity of 7,8-Dihydro-6H-thiopyrano[3,2-delta]pyrimidine derivatives and related compounds"

## Description

### BACKGROUND OF THE INVENTION

In the BE-A-724745 and in the corresponding FR-M-8437 there are disclosed certain 6H-7,8-dihydrothiapyrano[3,2-d]-pyrimidines as intermediates for the preparation of compounds with cardiovascular and coronary dilation activity. However, suggestion is made neither of any hypoglycemic activity nor of weight reducing properties for either the intermediates or the final products.

In the FR-A-2 522 000 and in the GB-A, B 2 119 368 respectively, there are disclosed 6H-7,8-dihydrothiapyrano[3,2-d]pyrimidines with a very different substitution pattern on the nucleus when compared with the compounds which are used according to the present invention as pharmaceutically effective ingredient for the preparation of pharmaceutical compositions for lowering the blood glucose levels and/or for effecting weight loss in obese patients.

It was now surprisingly found out that certain 6H-7,8-dihydrothiapyrano[3,2-d]-pyrimidines, including such compounds which are disclosed as intermediate products in BE-A-724745 and FR-M-8437 respectively, have a hypoglycemic and/or weight reducing activity. It, accordingly, was the object of the present invention to use said compounds as pharmaceutically effective ingredients for the preparation of corresponding pharmaceutical compositions.

### DESCRIPTION OF THE INVENTION

The object of the present invention is the use of compounds of formula I 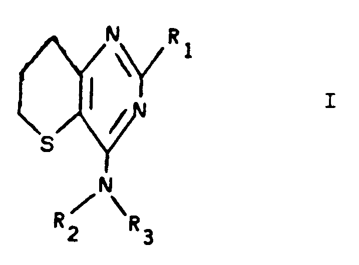
in which
- R₁ is: hydrogen or loweralkyl of from 1 to 6 carbon atoms;
- R₂ and R₃: are hydrogen or
- R₂ and R₃: form together with the nitrogen atom to which they are bonded a heterocycle of 5 members which may also include one or two additional heteroatoms independently selected from nitrogen, nitrogen substituted with lower alkyl of from 1 - 6 carbon atoms or oxygen or
- R₂ and R₃: form together with the nitrogen atom to which they are bonded a heterocycle of 6 members which may also include one additional heteroatom in the 2 or 3 position or 2 additional heteroatoms in any position independently selected from nitrogen, nitrogen substituted with lower alkyl of 1 - 6 carbon atoms or oxygen, or a single heteroatom may be in the 4 position selected from unsubstituted nitrogen or nitrogen substituted with loweralkyl of 1, 2, 3, 4, 5 or 6 carbon atoms,
or of pharmaceutically acceptable salts of said compounds as pharmaceutically effective ingredient for the preparation of pharmaceutical compositions for lowering the blood glucose levels and/or for effecting weight loss in obese patients.

The lower alkyl group of the compounds of formula I may contain from 1 to 6 carbon atoms and may be in either a straight or branched configuration. Exemplary of such groups are methyl, ethyl, propyl, isopropyl, butyl, sec-butyl, pentyl, hexyl, and the like.

The preferred compounds of formula I used according to the present invention are those wherein R₁ is methyl, ethyl, propyl or isopropyl; and R₂ and R₃ are joined to form the heterocyclic groups piperazine, N-methylpiperazine, N-ethylpiperazine, and N-propylpiperazine.

The instant compounds of formula I can be prepared from the appropriate R₁ substituted thiapyranopyrimidin-4-one which is treated with phosphorous oxychloride to prepare the analogous 4-chloro compound which, on treatment with the appropriately substituted amine or heterocyclic amine prepares the desired compounds. The general synthetic procedures are described in BE-A 724245.

Diabetes is a condition characterized by abnormal insulin secretion and a variety of metabolic and vascular manifestations reflected in a tendency toward inappropriately elevated blood glucose levels and which if left poorly treated or untreated can result in accelerated, nonspecific athersclerosis, neuropathy and thickened capillary lamina causing renal and retinal impairment. Diabetes is characterized as being insulin dependent (Type I) and non-insulin dependent (Type II). Type I diabetes is due to damage and eventual loss of the β-cells of the pancretic islets of Langerhans with a resulting loss of insulin production. Type II diabetics secrete insulin, however, the insulin is somehow not properly or effectively utilized in the metabolism of blood sugars and glucose accumulates in the blood to above normal levels. This condition is termed insulin resistance.

With the certainty of serious complications resulting from high glucose levels in poorly controlled or uncontrolled diabetics, means to lower blood glucose have been research goals for a considerable period of time. With Type I diabetes glucose control can only be achieved with daily insulin injections. With Type II diabetes glucose control can be effected from a combination of diet and drugs which lower glucose levels. The currently available oral hypoglycemic agents are not completely satisfactory since they may not offer complete blood glucose control or may provide a variety of undesirable side effects or they may elevate insulin concentrations to undesirable and dangerous levels. Thus, the search for improved oral hypoglycemic agents is a continuing one.

As previously indicated, the compounds used according to the present invention for the preparation of pharmaceutical compositions are all ready adapted to be used as hypoglycemic agents for the preparation of pharmaceutical compositions which are suited for oral administration. This is due to the ability of the compounds of formula I and salts thereof to lower the blood sugar levels of diabetic subjects to a statistically significant degree. For instance, 2-methyl-4-(4-methylpiperazine)6H-7,8-dihydro thiapyrano [3,2-d]pyrimidine, a typical and preferred agent used according to the present invention, has been found to consistently lower blood sugar levels and improve glucose tolerance in either fasted or fed diabetic (i.e., hyperglycemic) mice to a statistically significant degree when given by the oral route of administration at dose levels ranging from 1 mg/kg to 100 mg/kg, respectively, without showing any toxic side effects. The other compounds used according to the invention also produce similar results. In general, the corresponding compositions are ordinarily administered at dosage levels ranging from about 1 mg to about 100 mg of active ingredient per kg of body weight per day, although variations will necessarily occur depending upon the condition and individual response of the subject being treated and the particular type of oral pharmaceutical formulation chosen.

Administration over time to obese, insulin resistant mice, resulted in a significant reduction in body weight.

In connection with the inventive use of the compounds of formula I for the preparation of pharmaceutical compositions for the treatment of diabetic subjects, it is to be noted that the compositions may contain the active ingredient either alone or in combination with pharmaceutically acceptable carriers and that the administration can be carried out in both single and multiple dosages.More particularly, the compounds of formula I or salts thereof can be formulated to a wide variety of different dosage forms, i.e., they may be combined with various pharmaceutically acceptable inert carriers in the forms of e.g. tablets, capsules, lozenges, troches, hard candies, powders, aqueous suspension, elixirs and syrups. Such carriers include diluents or fillers, sterile aqueous media and various non-toxic organic solvents. Moreover, such oral pharmaceutical compositions can be suitably sweetened and/or flavored by means of various agents of the type commonly employed for just such a purpose. In general, the therapeutically-effective compounds of formula I or pharmaceutically acceptable salts thereof are present in such dosage forms at concentration levels ranging from about 0.5% to about 90% by weight of the total composition, i.e., in amounts which are sufficient to provide the desired unit dosage.

For purposes of oral administration, tablets containing various excipients such as sodium citrate, calcium carbonate and dicalcium phosphate may be employed along with various disintegrants such as starch and preferably potato or tapioca starch, alginic acid and certain complex silicates, together with binding agents such as polyvinylpyrrolidone, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred materials in this connection would also include the high molecular weight polyethylene glycols. When aqueous suspensions and/or elixirs are desired for oral administration, the essential active ingredient therein may be combined with various sweetening or flavoring agents, coloring matter or dyes and, if so desired, emulsifying and/or suspending agents as well, together with such diluents as water, ethanol, propylene glycol, glycerin and various like combinations thereof.

The activity of the compounds of formula I which are used according to the invention as hypoglycemic agents for the preparation of corresponding pharmaceutical preparations is determined by their ability to lower blood sugar levels in the fasted or fed hyperglycemic mouse when tested therein for such purposes according to the procedures described by Saperstein et al. as submitted to the journal Diabetes and summarized as follows: Genetically obese mice (ob/ob) were fasted overnight. The compounds were administered orally via a stomach tube and each mouse serially bled from the orbital sinus at various times and the blood samples were analyzed for blood glucose. When the effects of the compounds on blood glucose levels were to be determined, glucose was administered orally at a rate of 2 g per kg. 30 minutes after administration of the test compound. Glucose in the blood was determined by the potassium ferricyanide potassium ferrocyanide oxidation reaction auto analyzer. The latter method measures directly the amount of glucose in the blood at any given time and from this, the maximum percent decrease in blood sugar can be readily calculated and reported as hypoglycemic activity per se. In this way, the present compounds are shown to markedly improve glucose tolerance of non-anesthetized hyperglycemic mice when administered to them at dose levels as low as 10 mg/kg orally and lower fasting blood glucose levels when adminstered at dose levels as low as 30 mg/kg orally.

## Claims

1. Use of compounds of formula I 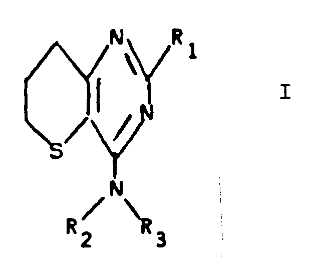 in which
R₁ is hydrogen or loweralkyl of from 1 to 6 carbon atoms;
R₂ and R₃ are hydrogen or
R₂ and R₃ form together with the nitrogen atom to which they are bonded a heterocycle of 5 members which may also include one or two additional heteroatoms independently selected from nitrogen, nitrogen substituted with lower alkyl of from 1 - 6 carbon atoms or oxygen or
R₂ and R₃ form together with the nitrogen atom to which they are bonded a heterocycle of 6 members which may also include one additional heteroatom in the 2 or 3 position or 2 additional heteroatoms in any position independently selected from nitrogen, nitrogen substituted with lower alkyl of 1 - 6 carbon atoms or oxygen, or a single heteroatom may be in the 4 position selected from unsubstituted nitrogen or nitrogen substituted with loweralkyl of 1, 2, 3, 4, 5 or 6 carbon atoms,
or of pharmaceutically acceptable salts of said compounds as pharmaceutically effective ingredient for the preparation of pharmaceutical compositions for lowering the blood glucose levels and/or for effecting weight loss in obese patients.

2. Use according to claim 1 characterized in that a compound of formula I is used in which
R₁ is methyl, ethyl, propyl or isopropyl; and
R₂ and R₃ are independently hydrogen or
R₂ and R₃ form together with the nitrogen atom to which they are bonded the heterocyclic group piperazine, N-methyl-piperazine, N-ethylpiperazine or N-propyl-piperazine.

3. Use according to claim 1 or 2, characterized in that for the preparation of the pharmaceutical compositions there is used as pharmaceutically effective ingredient of formula I the 2-methyl-4-(4-methylpiperazine)-6 H-7,8-dihydro thiapyrano [3,2-d]pyrimidine.

4. Use according to one of the claims 1- 3, characterized in that the compounds of formula I or pharmaceutically acceptable salts thereof, are used for the preparation of pharmaceutical compositions for oral administration.

5. Use according to one of the claims 1 - 4, characterized in that the active ingredients of formula I are used to prepare pharmaceutical compositions which contain 0,5 - 90 % by weight, referred to the pharmaceutical composition, of said ingredient and furthermore a pharmaceutically acceptable carrier material.

## Patentansprüche

1. Verwendung von Verbindungen der Formel I 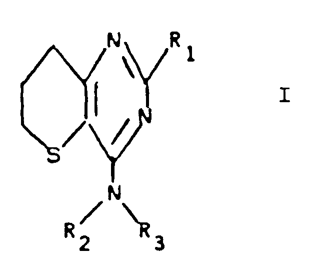 worin
R₁ Wasserstoff oder Niederalkyl mit 1 bis 6 Kohlenstoffatomen ist;
R₂ und R₃ Wasserstoff sind oder
R₂ und R₃ mit dem Stickstoffatom, an das sie gebunden sind, zusammen einen 5-gliedrigen Heterozyklus bilden, der auch ein oder zwei weitere Heteroatome enthalten kann, unabhängig ausgewählt aus Stickstoff, mit Niederalkyl von 1 bis 6 Kohlenstoffatomen ausgewähltem Stickstoff oder Sauerstoff, oder
R₂ und R₃ mit dem Stickstoffatom, an das sie gebunden sind, zusammen einen 6-gliedrigen Heterozyklus, der auch ein weiteres Heteroatom in der 2- oder 3-Stellung oder zwei weitere Heteroatome in beliebiger Stellung, unabhängig ausgewählt aus Stickstoff, mit Niederalkyl mit 1 bis 6 Kohlenstoffatomen substituiertem Stickstoff oder Sauerstoff, oder in der 4-Stellung ein einzelnes Heteroatom, ausgewählt aus unsubstituiertem Stickstoff oder mit Niederalkyl mit 1, 2, 3, 4, 5 oder 6 Kohlenstoffatomen substituiertem Stickstoff, enthalten kann,
oder pharmazeutisch annehmbarer Salze der Verbindungen als pharmazeutischer Wirkstoff für die Herstellung pharmazeutischer Zusammensetzungen zur Absenkung des Blutzuckerspiegels und/oder zur Herbeiführung eines Gewichtsverlusts in übergewichtigen Patienten.

2. Verwendung nach Anspruch 1, dadurch gekennzeichnet, daß eine Verbindung der Formel I verwandt wird, worin R₁ Methyl, Ethyl, Propyl oder Isopropyl ist und R₂ und R₃ unabhängig Wasserstoff sind oder R₂ und R₃ zusammen mit dem Stickstoffatom, an das sie gebunden sind, die heterozyklische Gruppe Piperazin, N-Methylpiperazin, N-Ethylpiperazin oder N-Propylpiperazin bilden.

3. Verwendung nach Anspruch 1 oder 2, dadurch gekennzeichnet, daß zur Herstellung der pharmazeutischen Zusammensetzung als pharmazeutischer Wirkstoff der Formel I 2-Methyl-4-(4-methylpiperazin)-6H-7,8-dihydrothiapyrano[3,2-d]-pyrimidin verwandt wird.

4. Verwendung nach einem der Ansprüche 1 bis 3, dadurch gekennzeichnet, daß die Verbindungen der Formel I oder pharmazeutisch annehmbare Salze davon zur Herstellung einer pharmazeutischen Zusammensetzung für die orale Verabreichung verwandt werden.

5. Verwendung nach einem der Ansprüche 1 bis 4, dadurch gekennzeichnet, daß die Wirkstoffe der Formel I eingesetzt werden, um pharmazeutische Zusammensetzungen herzustellen, die 0,5 bis 90 Gew.-%, bezogen auf die pharmazeutische Zusammensetzung, Wirkstoff und weiterhin ein pharmazeutisch annehmbares Trägermaterial enthalten.

## Revendications

1. Utilisation des composés de formule I 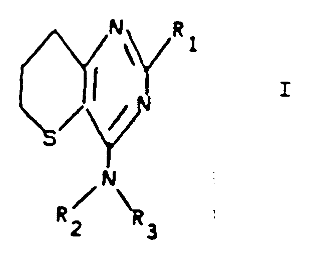 dans laquelle
R₁ est un hydrogène ou un alkyle inférieur ayant de 1 à 6 atomes de carbone;
R₂ et R₃ sont de l'hydrogène ou
R₂ et R₃ forment conjointement avec l'atome d'azote auquel ils sont liés un hétérocycle à 5 membres qui peut aussi inclure un ou deux hétéroatomes additionnels indépendamment choisis parmi un azote, un azote substitué avec un alkyle inférieur ayant de 1 à 6 atomes de carbone ou un oxygène ou
R₂ et R₃ forment conjointement avec l'atome d'azote auquel ils sont liés un hétérocycle à 6 membres qui peut aussi inclure un hétéroatomes additionnel en position 2 ou 3 ou 2 hétéroatomes additionnels en n'importe quelle position indépendamment choisis parmi un azote, un azote substitué avec un alkyle inférieur ayant de 1 à 6 atomes de carbone ou un oxygène, ou un unique hétéroatome peut être en position 4 choisi parmi un azote non substitué ou un azote substitué avec un alkyle inférieur ayant 1, 2, 3, 4, 5 ou 6 atomes de carbone,
ou des sels pharmaceutiquement acceptables desdits composés en tant qu'ingrédient pharmaceutiquement efficace pour la préparation de compositions pharmaceutiques pour abaisser les taux en glucose sanguin et/ou pour être des effecteurs d'une perte de poids chez des patients obèses.

2. Utilisation selon la revendication 1 caractérisée en ce qu'un composé de formule I est utilisé dans lesquels R₁ est un méthyle, éthyle, propyle ou isopropyle; et R₂ et R₃ sont indépendamment de l'hydrogène ou R₂ et R₃ forment conjointement avec l'atome d'azote auquel ils sont liés un groupement hétérocyclique pipérazine, N-méthyle-pipérazine, N-éthyle-pipérazine, ou N-propyle-pipérazine.

3. Utilisation selon la revendication 1 ou 2,caractérisée en ce que pour la préparation des compositions pharmaceutiques on utilise comme ingrédient pharmaceutiquement efficace de formule I la 2-méthyl-4-(4-méthylpipérazine)-6H-7,8-dihydro thiapyrano [3,2-d]pyrimidine.

4. Utilisation selon l'une des revendications 1-3, caractérisée en ce que les composés de formule I ou des sels pharmaceutiquement acceptables de ceux-ci, sont utilisés pour la préparation de compositions pharmaceutiques pour une administration orale.

5. Utilisation selon l'une des revendications 1-4, caractérisée en ce que les ingrédients actifs de formule I sont utilisés pour préparer des compositions pharmaceutiques qui contiennent de 0,5 à 90% en masse, par référence à la composition pharmaceutique, dudit ingrédient et au surplus un matériau de support pharmaceutiquement acceptable.
